# EUROPEAN PATENT SPECIFICATION

(11) **EP 4 496 582 B1**
(45) Date of publication and mention of the grant of the patent: **21.10.2026**
(21) Application number: 23712914.3
(22) Date of filing: 22.03.2023
(51) Int. Cl.: A61K 38/20, C07K 16/28, A61P 35/00

(54) **L19-IL2 IMMUNOCONJUGATE PREPARATION AND ITS USE FOR TREATING A CANCER**
L19-IL2-IMMUNOKONJUGAT-PRÄPARAT UND SEINE ANWENDUNG ZUR BEHANDLUNG VON KREBS
PRÉPARATION D'IMMUNOCONJUGUÉ L19-IL2 ET SON UTILISATION DANS LE TRAITEMENT DU CANCER

(30) Priority: 23.03.2022 EP 22163911
(43) Date of publication of application: 29.01.2025
(73) Proprietor: Philogen S.p.A., 53100 Siena (IT)
(72) Inventor: BATTESTIN, Paola, 53100 Siena (IT); BACCI, Camilla, 53100 Siena (IT); REDI, Alessandro, 53100 Siena (IT); SANI, Emiliano, 53100 Siena (IT); GILARDONI, Ettore, 8112 Otelfingen (CH); STUCCHI, Riccardo, 8112 Otelfingen (CH); DE LUCA, Roberto, 8112 Otelfingen (CH); DI NITTO, Cesare, 8112 Otelfingen (CH); MATASCI, Mattia, 8112 Otelfingen (CH); NERI, Dario, 53100 Siena (IT)
(74) Representative: Mewburn Ellis LLP
(86) International application number: PCT/EP2023/057391
(87) International publication number: WO 2023/180409

(56) References cited:
- EP-A1- 2 085 095
- WO-A1-2019/185792
- SCHLIEMANN CHRISTOPH ET AL: "Complete eradication of human B-cell lymphoma xenografts using rituximab in combination with the immunocytokine L19-IL2", BLOOD, AMERICAN SOCIETY OF HEMATOLOGY, US, vol. 113, no. 10, 1 March 2009 (2009-03-01), pages 2275 - 2283, XP009139092, ISSN: 0006-4971, [retrieved on 20081112], DOI: 10.1182/BLOOD-2008-05-160747

## Description

This application claims priority from European Patent Application No. 22163911.5 filed 23 March 2022.

### Field of the Invention

The present invention relates to a preparation, in particular a pharmaceutical preparation, comprising a non-glycosylated IL2 immunoconjugate and a glycosylated IL2 immunoconjugate, wherein the percentage of the glycosylated IL2 immunoconjugate of the total IL2 immunoconjugate in the preparation is low, i.e., 20% or lower. The preparation has been shown to have an increased T cell activation activity relative to a preparation with a higher percentage of the glycosylated IL2 immunoconjugate. The preparation finds application in the treatment of cancer in human patients.

### Background

Current applicants described in WO2001/062298, an immunoconjugates comprising human IL2 fused to antibody L19 ("L19-IL2"). L19 (US patent n° 8,097,254) specifically binds the ED-B domain of fibronectin isoform B-FN, which is one of the best-known markers of angiogenesis. ED-B is an extra domain of 91 amino acids found in the B-FN isoform. ED-B accumulates around neovascular structures in aggressive tumours and other tissues undergoing angiogenesis, such as the endometrium in the proliferative phase and some ocular structures in pathological conditions but is otherwise undetectable in normal adult tissues. A number of uses of L19-IL2 have been previously described by the current applicants in WO2007/115837, WO2009/089858, WO2013/010749, WO2013/045125, WO2018/154517, WO2019/185792, WO2020/070150, in particular its use in the treatment of human cancers.

One difficulty with the production L19-IL2 for administration to human patients, is the extensive glycosylation when IL2 is produced in eukaryotic cells. Specifically, production of human IL2 in Chinese Hamster Ovary (CHO) cells had previously been shown to result in high levels of glycosylation, with glycosylated IL2 vastly exceeding non-glycosylated IL2 (Ferrara *et al.,* FEBS Letters (1987), 226, 47). US patent no. 5,417,970 similarly reported that expression of recombinant human IL2 in CHO cells resulted in about 50% of the total IL2 present in the preparation being glycosylated.

High levels of glycosylation present challenges in terms of batch-to-batch reproducibility for good manufacturing practice (GMP) production, increased immunogenicity and suboptimal pharmacokinetics when preparing a product for industrial development. Durocher & Butler, Current Opinion Biotechnol (2009), 20, 770 explain that glycan structures may be immunogenic and that immunogenicity can reduce the efficacy of a biologic through rapid clearance by immune system. Planinc et al. Analytica Chimica Acta (2016) 13, 27 similar confirm the importance of low abundance of glycans due to their potential immunogenicity. Leepenies & Seeberger Nature Biotechnol. (2016), 32, 443 further comment that heterogeneous glycosylation can result in batch-to-batch variations in efficacy or pharmacokinetics.

Although, theoretically, non-glycosylated proteins can be obtained through recombinant expression in prokaryotic cells, this is not possible in the context of complex proteins such as L19-IL2, which forms a large 80 KDa non-covalent homodimer in solution.

Despite the above-referenced immunogenicity and GMP compliance challenges associated with high levels of glycosylation, the art considers high levels of glycosylation in the IL2 molecule as advantageous for promoting T cell proliferation. For example, Pawelec *et al.* Immunobiology (1987), 174, 67, compared glycosylated IL2 purified from natural sources with non-glycosylated IL2 produced in *E. Coli* and concluded that for propagation of human T cells, glycosylated IL2 is preferable to non-glycosylated IL2 (see e.g. abstract).

In light of the above, there remains a need in the art for preparations of L19-IL2 having a favourable profile both in terms of (i) immunogenicity and (ii) T cell activation activity, e.g. for use in the treatment of cancer.

### Summary of the Invention

The present invention is defined by the claims.

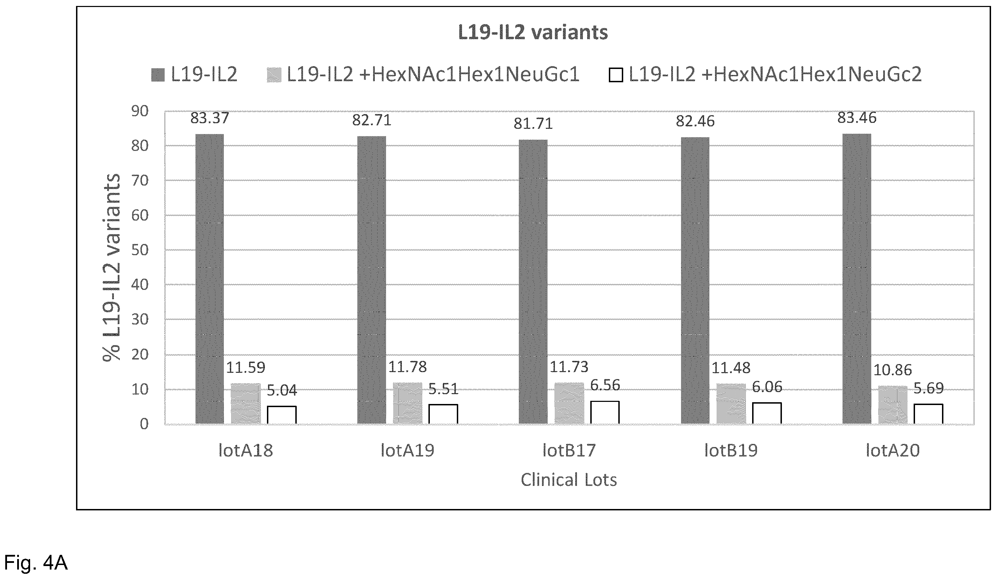

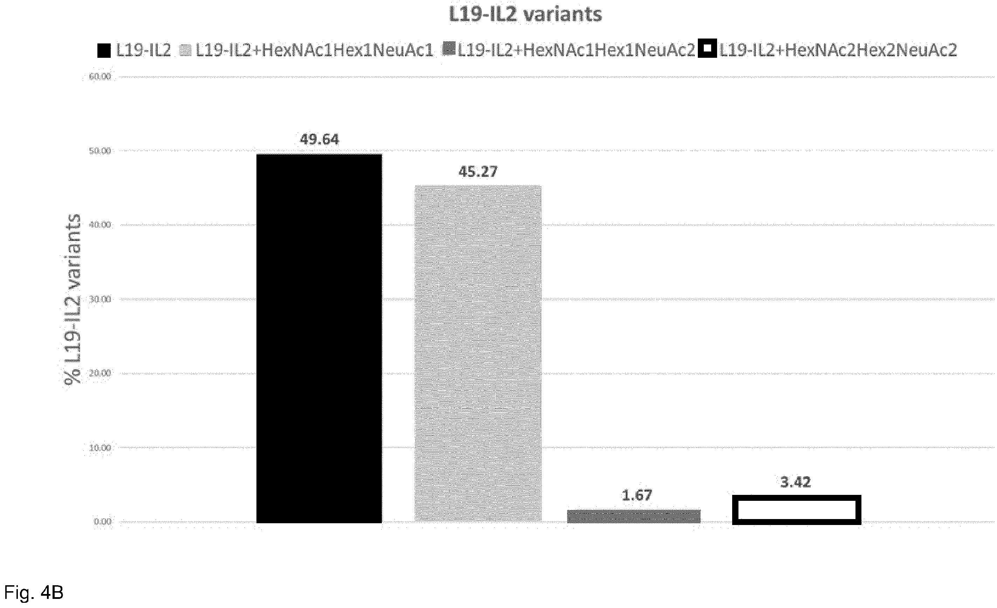

The present inventors have devised a production method for the preparation of L19-IL2, which results in a high yield and, surprisingly, a low percentage of glycosylated L19-IL2 in the resulting preparation. Specifically, the percentage of glycosylated L19-IL2 in the preparation was reported to be between about 16.5% and 18.5%, with an average of 17% of the L19-IL2 being glycosylated **(****Figure 4****).** In view of the high rates of glycosylation reported in the literature when IL2 was produced in eukaryotic cells (see discussion above), this was highly surprising. Previously described methods for producing the L19-IL2 conjugate had not provided any information on glycosylation levels (Carnemolla et. al. Blood (2002) 99, 1659).

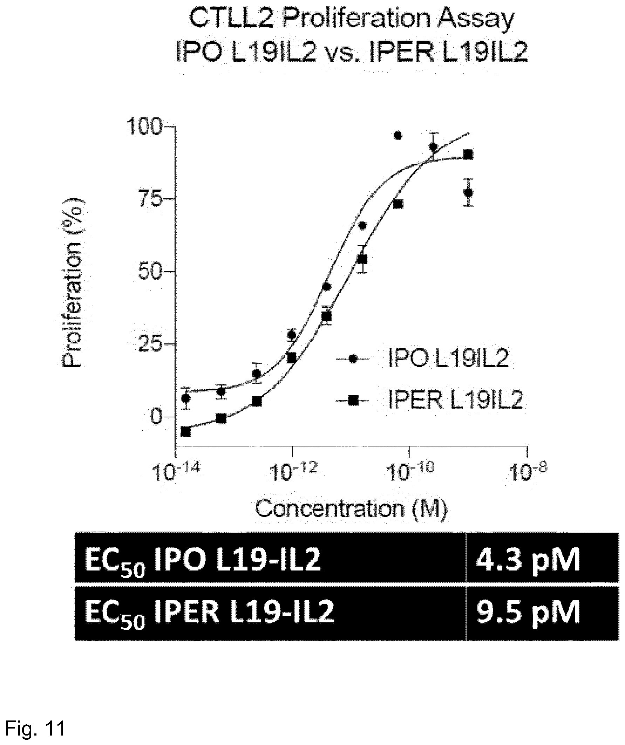

Furthermore, when preparations comprising a low percentage of glycosylated L19-IL2 ("I PO L19-IL2") were compared with preparations comprising a high percentage of glycosylated L19-IL2 ("IPER L19-IL2"), the preparations with a low percentage of glycosylated L19-IL2 ("IPO L19-IL2") unexpectedly resulted in superior T cell activation compared with preparations comprising a high percentage of glycosylated L19-IL2 ("IPER L19-IL2") (Figure 11). This was highly surprising in view of the literature which reported that a glycosylation of IL2 resulted in superior T cell activation (see discussion above).

Without wishing to be bound by theory, it is believed that the fermentation conditions used to prepare the L19-IL2 conjugate described herein result in the low level of glycosylated species observed. In particular, the inventors were able to show that differences in downstream processes and process parameters did not influence percentage of glycosylated L19-IL2 present in the final preparation.

In view of the above, the low percentage of glycosylated L19-IL2 in the preparation resulting from the production method devised by the present inventors is expected to have advantageous properties when used in the treatment of human patients compared with L19-IL2 preparations comprising higher percentages of glycosylated L19-IL2. The advantageous properties include an increased T cell activation activity.
20%

Thus, in a first embodiment, the present invention provides a preparation comprising:
(i) non-glycosylated L19-IL2; and
(ii) glycosylated L19-IL2;

wherein the L19-IL2 comprises or consists of the sequence set forth in SEQ ID NO: 13; and
wherein the percentage of glycosylated L19-IL2 is ≤ 20% of the total L19-IL2 in the preparation.

The percentage of glycosylated IL2 immunoconjugate of the total IL2 immunoconjugate in the preparation is preferably ≤ 19%, or ≤ 18.5%, with a lower % of glycosylation being preferred for the reasons explained above.

The percentage of glycosylated IL2 immunoconjugate of the total IL2 immunoconjugate in the preparation may be ≥ 2%, ≥ 5%, ≥ 10%, or ≥ 15%.

For example, the percentage of glycosylated IL2 immunoconjugate of the total IL2 immunoconjugate in the preparation may be ≥ 2% and ≤ 20%.

The glycosylated IL2 immunoconjugate comprises an *O*-linked glycosylation at the threonine at position 3 of IL2, wherein the IL2 has the sequence set forth in SEQ ID NO: 11. Where the IL2 immunoconjugate is L19-IL2 and comprises or consists of the sequence set forth in SEQ ID NO: 13, the glycosylated L19-IL2 preferably comprises an *O*-linked glycosylation on the threonine at position 256 of SEQ ID NO: 13. The *O*-linked glycosylation may be linear or branched. In the IL2 immunoconjugate with a linear *O*-linked glycosylation, the *O*-linked glycosylation preferably comprises one core of HexoseN-acetyl-Hexose1 plus 1 N-glycolyl neuraminic acid, while in the IL2 immunoconjugate comprising a branched *O*-linked glycosylation, the *O*-linked glycosylation preferably comprises one core of HexoseN-acetyl-Hexose1 plus 2 N-glycolyl neuraminic acid residues. More preferably, the *O*-linked glycosylation is HexNAc₁Hex₁NeuGc₁ or HexNAc₁Hex₁NeuGc₂. In one example, the *O*-linked glycosylation is Neu5Gcα2-3Galβ1-3GalNAcC1S(3)1 or Neu5Gcα2-3Galβ1-3(Neu5Gcα2-6)GalNAcC1S(3,6)2.

In a preferred embodiment, the IL2 immunoconjugate is not glycosylated at any other position in the IL2 sequence.

In one embodiment, the present invention provides a preparation comprising:
(i) a non-glycosylated IL2 immunoconjugate; and
(ii) a glycosylated IL2 immunoconjugate;

wherein the L19-IL2 comprises or consists of the sequence set forth in SEQ ID NO: 13,
wherein the percentage of glycosylated L19-IL2 is ≤ 20% of the total L19-IL2 in the preparation, and
wherein the preparation has a higher T cell activation activity compared to a preparation in which the percentage of glycosylated L19-IL2 is 45% or more, preferably 45%, of the total L19-IL2 in the preparation.

Methods for determining the type and percentage of glycosylated IL2 immunoconjugate in a preparation of the IL2 immunoconjugate are known to the skilled person and include mass spectrometry, in particular intact mass analysis, as described herein.

The pharmaceutical preparation of the invention may further comprise, in addition to the active ingredient (glycosylated and non-glycosylated IL2 immunoconjugate), a pharmaceutically acceptable excipient, carrier, buffer, stabiliser or other materials well known to those skilled in the art and suitable for the administration to human patients. Such materials should be non-toxic and should not interfere with the efficacy of the active ingredient. For injection at a tumour site, the pharmaceutical preparation may be in the form of a parenterally acceptable aqueous solution which is pyrogen-free and has suitable pH, isotonicity and stability

The pharmaceutical preparation of the invention may be for use in a method of treating cancer in a patient. Also provided is a method of treating cancer, the method comprising administering a therapeutically effective amount of the pharmaceutical preparation of the invention to the patient. Similarly provided is the use of the pharmaceutical preparation of the invention in the manufacture of a medicament for the treatment of cancer. The patient is preferably a human patient.

Cancers that may be treated using a pharmaceutical preparation of the invention include solid cancers, such as skin cancer, renal cancer, pancreatic cancer, non-small cell lung cancer, diffuse large B cell lymphoma (DLBCL), basal cell carcinoma, or cutaneous squamous cell carcinoma.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

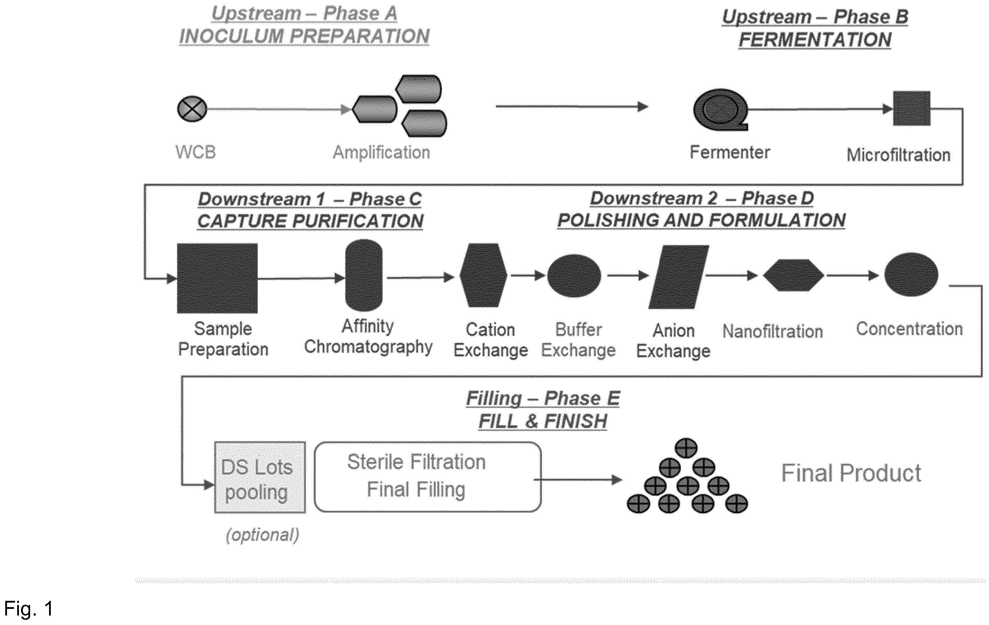

**Figure 1** shows the general outline of the production process of L19-IL2 used to obtain the pharmaceutical preparation comprising a low percentage of glycosylated L19-IL2 of the invention.

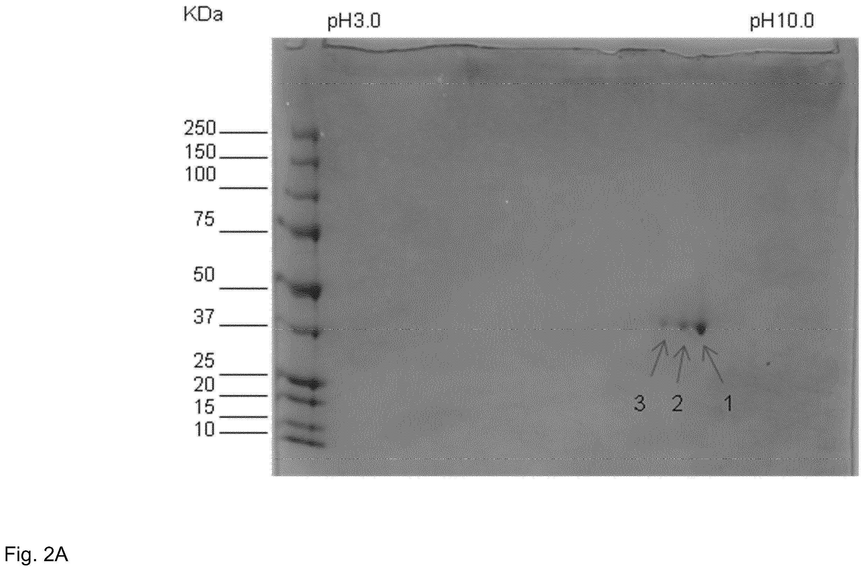

**Figure 2A****:** bidimensional SDS-PAGE analysis of the pharmaceutical preparation comprising a low percentage of glycosylated L19-IL2 confirmed the purity of the sample after production, as well as the molecular weight (~42 KDa) and isoelectric point (~7.7) of L19-IL2. Three spots were visible: non-glycosylated L19-IL2 (spot 1), mono-glycosylated L19-IL2 (spot 2) and di-glycosylated L19-IL2 (spot 3). Spot 1 (non-glycosylated L19-IL2) was the most intense, while spots 2 and 3 were barely visible, indicating the very low abundance of glycosylated L19-IL2 in the preparation. **B:** Purified milligrams of L19-IL2 per litre of fermentation culture recovered from five lots of production.

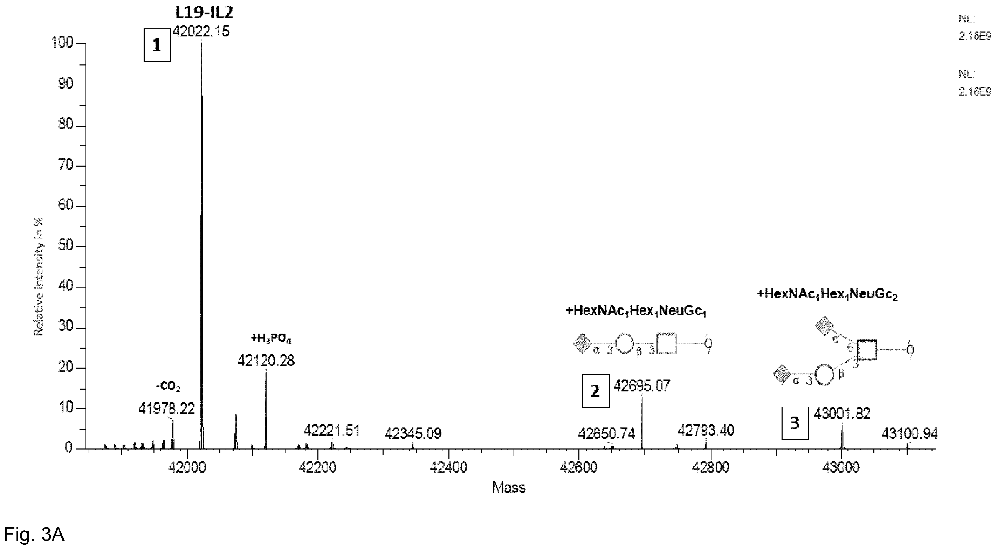

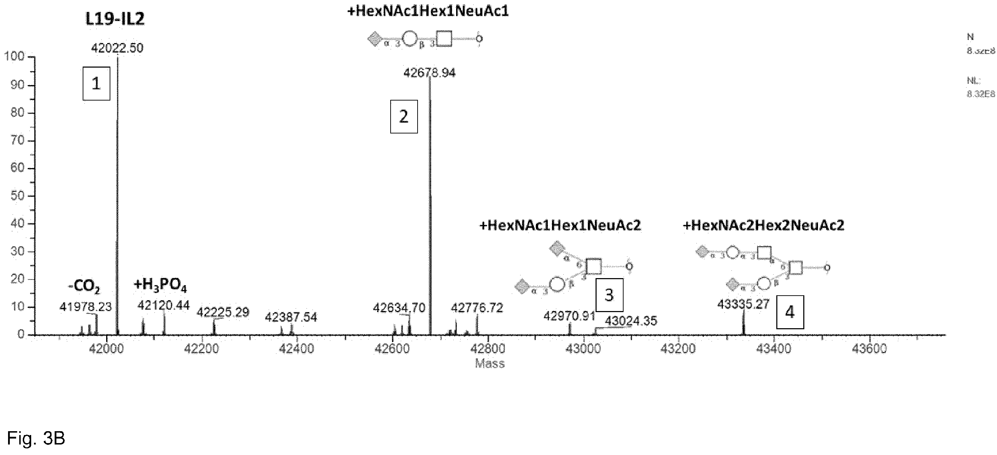

**Figure 3** **A:** shows the deconvoluted spectrum of the mass spectrometry analysis of a L19-IL2 preparation comprising a low percentage of glycosylated L19-IL2 prepared according to the method described herein. The main peak (1) at 42022 Da corresponds to the non-glycosylated L19-IL2 protein, while peak (2) at 42695 Da corresponds to the L19-IL2 variant with a glycan HexNAc1Hex1NeuGc1 and peak (3) at 43001 Da corresponds to the L19-IL2 variant with a glycan HexNAc1Hex1 NeuGc2. Other minor peaks are irrelevant: the small peak at 41978 Da is a typical MS artefact corresponding to L19-IL2 without one CO₂ group. The other small peak at 42120 Da corresponds to L19-IL2 coupled with a phosphate group of the buffer. **B:** (not according to the claimed invention; included for comparative purposes only) shows the deconvoluted spectrum of the mass spectrometry analysis of a L19-IL2 preparation comprising a high percentage of glycosylated L19-IL2. The peak (1) at 42022 Da corresponds to the non-glycosylated L19-IL2 protein, while peak (2) at 42678 Da corresponds to the L19-IL2 variant with a glycan HexNAc1Hex1NeuAc1, peak (3) at 42970 Da corresponds to the L19-IL2 variant with a glycan HexNAc1Hex1 NeuAc2, peak (4) at 4335 corresponds to the L19-IL2 variant with a glycan HexNAc2Hex2NeuAc2. Other minor peaks are irrelevant: the small peak at 41978 Da is a typical MS artefact corresponding to L19-IL2 without one CO₂ group. The other small peak at 42120 Da corresponds to L19-IL2 coupled with a phosphate group of the buffer.
**Figure 4** **A:** shows the percentage of non-glycosylated L19-IL2, mono-glycosylated L19-IL2 and di-glycosylated L19-IL2 of the total L19-IL2 in different production lots of the pharmaceutical preparation prepared using the method described herein. In five lots of production using the method described herein, glycosylated L19-IL2 never exceeded 20% of the total L19-IL2 in the preparation, with an average percentage of glycosylated L19-IL2 in the preparation of 17,06%. **B:** shows the percentage of non-glycosylated L19-IL2 and glycosylated L19-IL2 produced according to a standard protocol (i.e. not according to the claimed invention; included for comparative purposes only). In this case, non-glycosylated L19-IL2 and glycosylated L19-IL2 each formed 45-50% of the total L19-IL2 in the preparation.

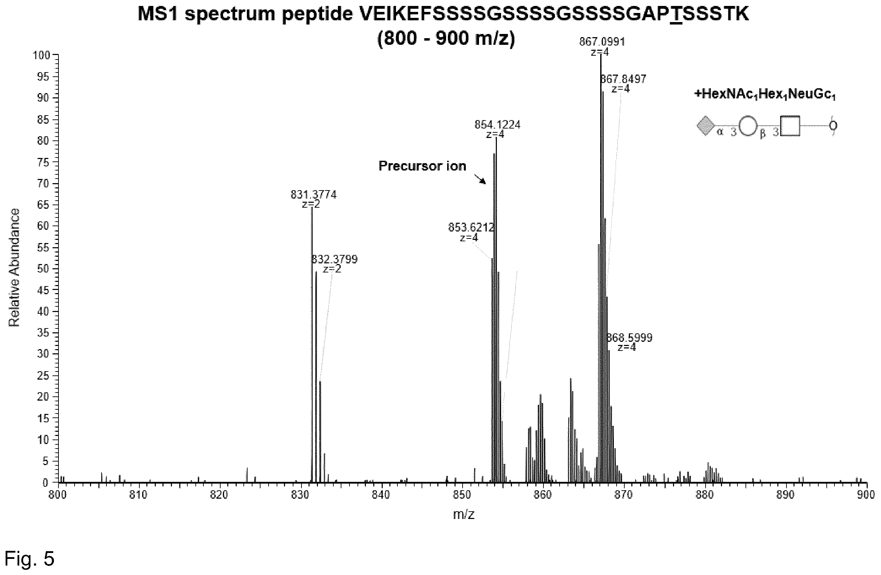

**Figure 5** shows the MS1 spectrum of the glycosylated peptide VEIKEFSSSSGSSSSGSSSSGAPT(+HexNAc1Hex1NeuGc1)SSSTK (SEQ ID NO:15) in a representative lot of production of a L19-IL2 preparation comprising a low percentage of glycosylated L19-IL2. Detection of a 4+ charged ion at 853.6212 m/z is consistent with the presence of one additional NeuGc group on the HexNAc1Hex1 core attached on the threonine at position 24 of the peptide, which corresponds to the threonine at position 256 in the L19-IL2 conjugate.

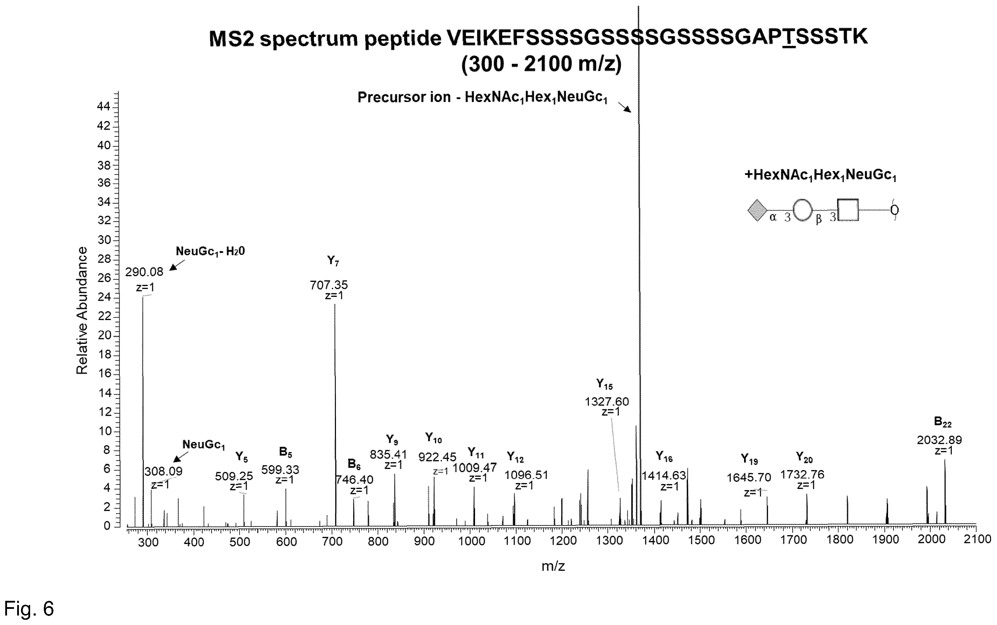

**Figure 6** shows the MS2 spectrum of the glycosylated peptide VEIKEFSSSSGSSSSGSSSSGAPT(+HexNAc1Hex1NeuGc1)SSSTK (SEQ ID NO:15) in a representative lot of production of a L19-IL2 preparation comprising a low percentage of glycosylated L19-IL2. The precursor ion at 853.62 m/z was isolated and HCD fragmented to generate this MS2 spectrum. Together with the expected list of y-ions, the presence of two peaks at 290.08 m/z (NeuGc1-H2O) and 308.09 m/z (NeuGc1) confirms the presence of the NeuGc group on the peptide. This data further confirms the correct identification of the HexNAc1Hex1NeuGc1 group on threonine at position 24 of the peptide, which corresponds to the threonine at position 256 in the L19-IL2 conjugate.

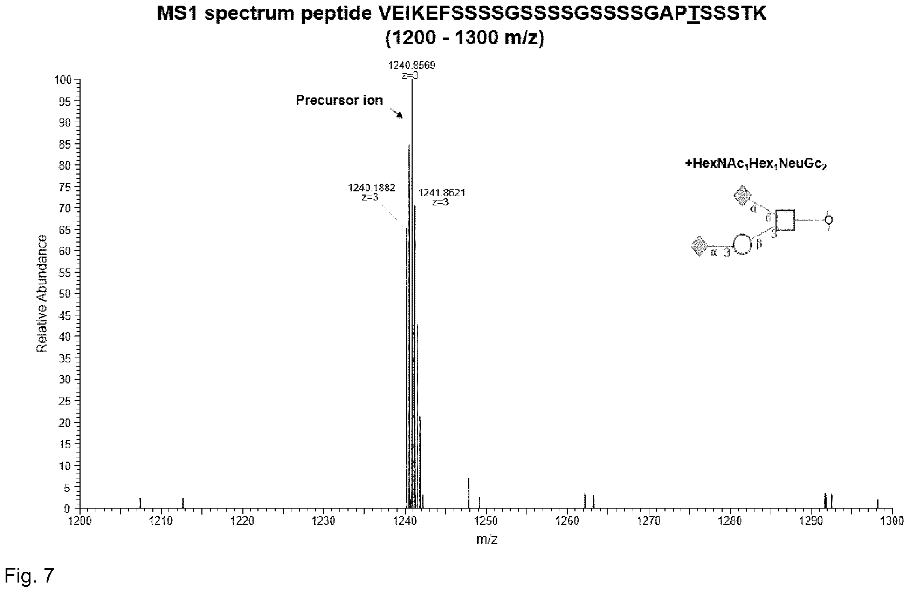

**Figure 7** shows the MS1 spectrum of the glycosylated peptide VEIKEFSSSSGSSSSGSSSSGAPT(+HexNAc1Hex1NeuGc2)SSSTK (SEQ ID NO:16) in a representative lot of production of a L19-IL2 preparation comprising a low percentage of glycosylated L19-IL2. Detection of a 3+ charged ion at 1240.1882 m/z is consistent with the presence of two additional NeuGc groups on the HexNAc1Hex1 core attached on the threonine at position 24 of the peptide, which corresponds to the threonine at position 256 in the L19-IL2 conjugate.

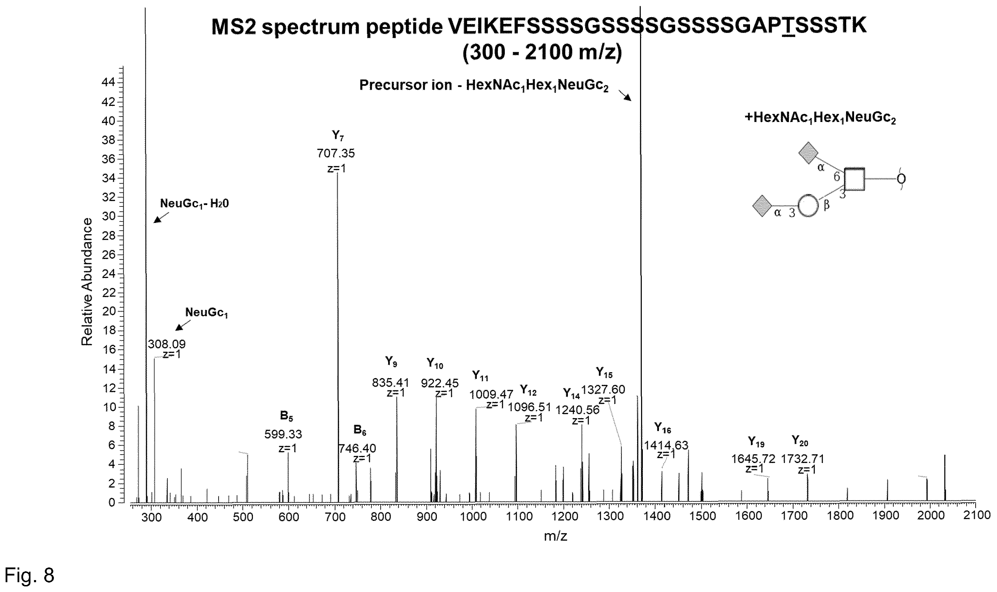

**Figure 8** shows the MS2 spectrum of the glycosylated peptide VEIKEFSSSSGSSSSGSSSSGAPT(+HexNAc1Hex1NeuGc2)SSSTK (SEQ ID NO:16) in a representative lot of production of a L19-IL2 preparation comprising a low percentage of glycosylated L19-IL2. The precursor ion at 1240.18 m/z was isolated and HCD fragmented to generate this MS2 spectrum. Together with the expected list of y-ions, the presence of two peaks at 290.08 m/z (NeuGc1-H2O) and 308.09 m/z (NeuGc1) confirms the presence of additional NeuGc groups on the peptide. This data further confirms the correct identification of the HexNAc1Hex1NeuGc2 group on the threonine at position 24 of the peptide, which corresponds to the threonine at position 256 in the L19-IL2 conjugate.

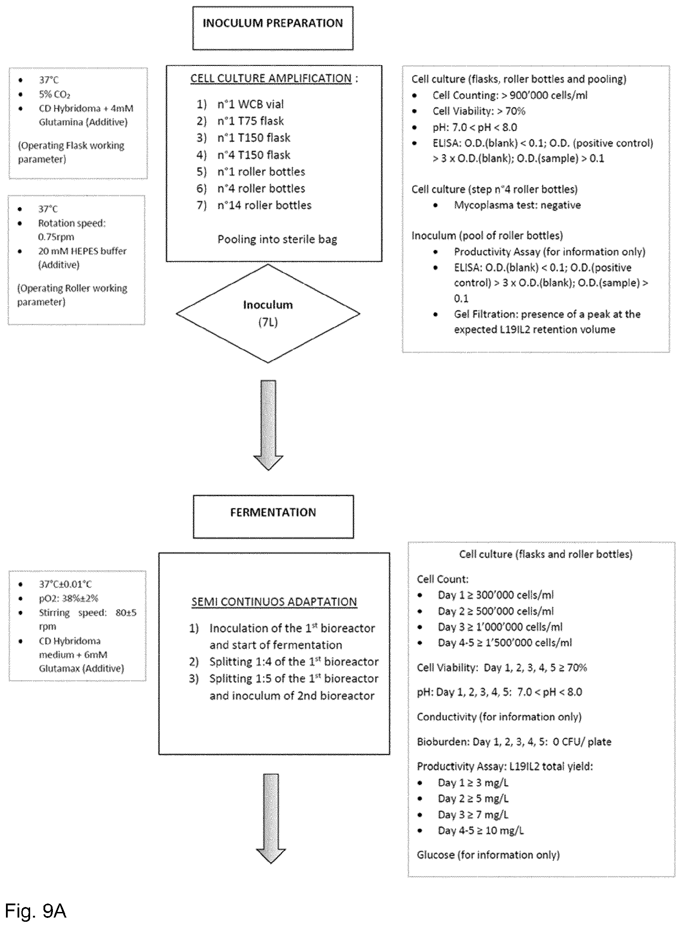

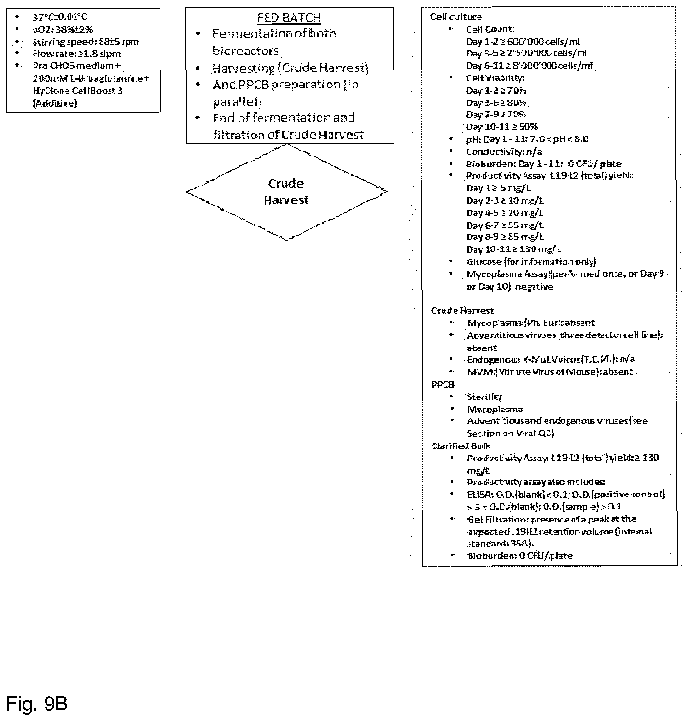

**Figures** 9A and B show a diagram describing the inoculum preparation and fermentation processes used to obtain a L19-IL2 preparation comprising a low percentage of glycosylated L19-IL2 (Upstream 1 and Upstream 2).

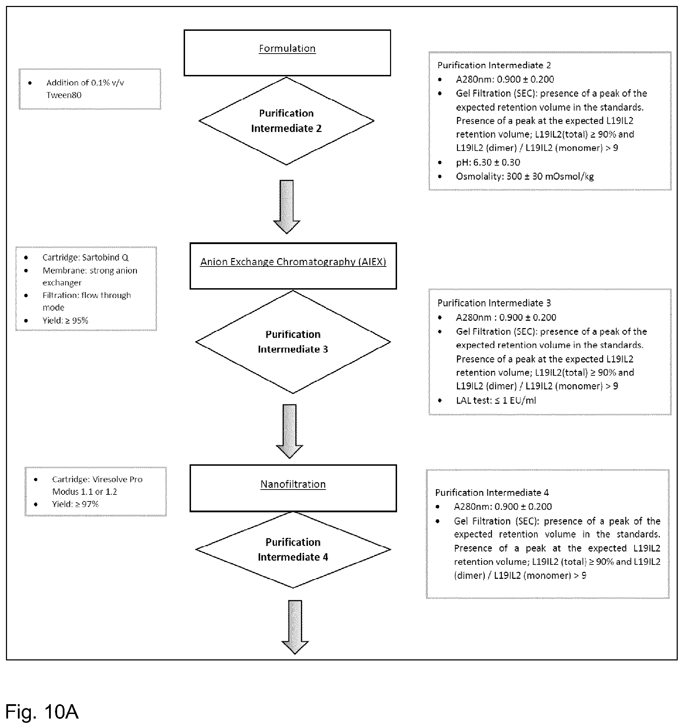

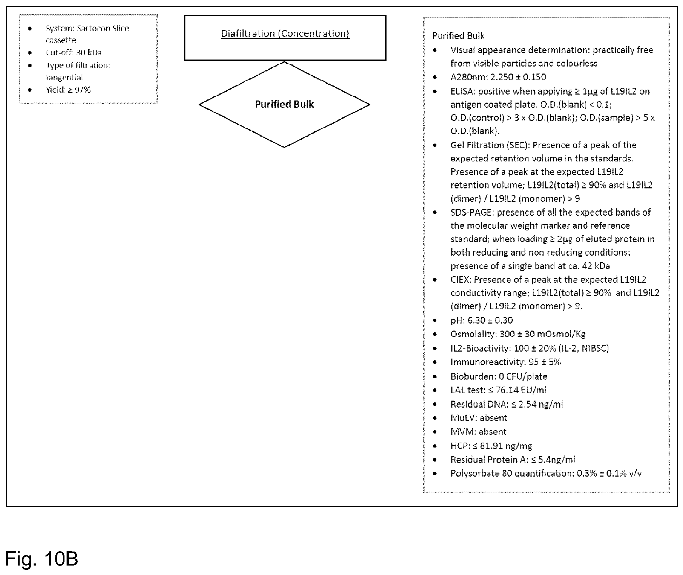

Figures 10A and B show a diagram describing the purification process steps used to obtain a L19-IL2 preparation comprising a low percentage of glycosylated L19-IL2 (Downstream 1 and Downstream 2).
Figure 11 shows a T cell activation assay comparing a L19-IL2 preparation comprising a low percentage of glycosylated L19-IL2 ("IPO L19IL2") according to the invention with a L19-IL2 preparation comprising a high percentage of glycosylated L19-IL2 ("IPER L191L2") prepared using standard methods. This demonstrates that a L19-IL2 preparation comprising a low percentage of glycosylated L19-IL2 shows superior T cell activation activity (EC50 4.3 pM) as compared to the L19-IL2 preparation comprising a high percentage of glycosylated L19-IL2 (EC50 9.5 pM).

### Detailed Description

Aspects and embodiments of the present invention will now be discussed with reference to the accompanying figures. Further aspects and embodiments will be apparent to those skilled in the art.

The pharmaceutical composition of the present invention comprises an IL2 immunoconjugate, as claimed, wherein the IL2 immunoconjugate comprises an antibody molecule comprising the L19 complementarity determining regions (CDRs) set forth in SEQ ID NOs 1 to 6 and IL2.

The antibody molecule is preferably monoclonal. The antibody molecule may be human or humanised, but preferably is a human antibody molecule.

The antibody molecule may be isolated, in the sense of being free from contaminants, such as antibodies able to bind other polypeptides, and/or serum components.

The antibody molecule may be natural or partly or wholly synthetically produced. For example, the antibody molecule may be a recombinant antibody molecule.

The antibody molecule may be an immunoglobulin, or an antigen-binding fragment thereof. For example, the antibody molecule may be an IgG, IgA, IgE or IgM molecule, preferably an IgG molecule, such as an IgG1, IgG2, IgG3 or IgG4 molecule, but more preferably is an antigen-binding fragment thereof. In a more preferred embodiment, the antibody molecule comprises or consists of a single-chain Fv (scFv), a small immunoprotein, a diabody, but most preferably is an scFv.

The antibody molecule comprises the L19 VH domain set forth in SEQ ID NO: 7 and/or the L19 VL domain set forth in SEQ ID NO: 9. Furthermore, the antibody molecule has the sequence of the L19 antibody in scFv format set forth in SEQ ID NO: 10.

Where the antibody molecule is an scFv, the VH and VL domains of the antibody are linked by a acid linker, wherein the linker sequence is set forth in SEQ ID NO: 8.

The IL2 is preferably human IL2. Furthermore, the IL2 has the sequence set forth in SEQ ID NO: 11.

The antibody molecule, e.g. scFv or IgG, and the IL2 may be connected to each other directly, for example through any suitable chemical bond, but preferably are connected via a peptide linker. The chemical bond may be, for example, a covalent or ionic bond. Examples of covalent bonds include peptide bonds (amide bonds) and disulphide bonds.

Where the IL2 is connected to the antibody molecule via a peptide linker, the peptide linker may be a short (2-30, preferably 10-20) residue stretch of amino acids. Suitable examples of peptide linker sequences are known in the art. One or more different linkers may be used. An exemplary linker sequence is set forth in SEQ ID NO 12. In one embodiment, the linker may be a cleavable linker.

Where the antibody molecule and IL2 are connected via a peptide bond or peptide linker, the conjugate may be produced (secreted) as a single chain polypeptide, such as a fusion protein. In a preferred embodiment, the IL2 is conjugated to the C-terminus of the antibody molecule in scFv format. Most preferably, the IL2 immunoconjugate consists of, the sequence of L19-IL2 set forth in SEQ ID NO: 13.

The IL2 in the glycosylated IL2 immunoconjugate comprised in the pharmaceutical preparation of the invention, preferably comprises an *O*-linked glycosylation on the threonine at position 3 of the IL2 sequence set forth in SEQ ID NO: 11. Where the IL2 immunoconjugate is L19-IL2 and comprises or consists of the sequence set forth in SEQ ID NO: 13, the glycosylated L19-IL2 preferably comprises an *O*-linked glycosylation on the threonine at position 256 of SEQ ID NO: 13. In a preferred embodiment, the glycosylated IL2 immunoconjugate is not glycosylated at any other position in the IL2 sequence.

The IL2 in the glycosylated IL2 immunoconjugate may be mono-glycosylated or di-glycosylated. In the mono-glycosylated IL2 immunoconjugate, the *O*-linked glycosylation is preferably HexNAc₁Hex₁NeuGc₁, while in the di-glycosylated IL2 immunoconjugate, the *O*-linked glycosylation is preferably HexNAc₁Hex₁NeuGc₂. The pharmaceutical preparation of the invention will usually comprise both mono-glycosylated IL2 immunoconjugate and di-glycosylated IL2 immunoconjugate, in addition to non-glycosylated IL2 immunoconjugate.

The L19 in the glycosylated L19-IL2 immunoconjugate comprised in the pharmaceutical preparation of the invention is not glycosylated. That is, only the IL2 in the glycosylated L19-IL2 immunoconjugate comprised in the pharmaceutical preparation of the invention is glycosylated. The percentage of glycosylated IL2 immunoconjugate of the total IL2 immunoconjugate in the pharmaceutical preparation of the invention is preferably ≤ 19.5%, ≤ 19%, or ≤ 18.5%, with a lower % of glycosylation being preferred for the reasons explained above. In the claimed embodiment, the % of glycosylation is ≤ 20%. In an alternative preferred embodiment, the % of glycosylation is ≤ 19.5%. In a further alternative preferred embodiment, the % of glycosylation is ≤ 19%. In a yet further alternative preferred embodiment, the % of glycosylation is ≤ 18.5%. The percentage of glycosylated IL2 immunoconjugate of the total IL2 immunoconjugate in the preparation may be ≥ 2%, ≥ 3%, ≥ 4%, ≥ 5%, ≥ 6%, ≥ 7%, ≥ 8%, ≥ 9%, ≥ 10%, ≥ 11%, ≥ 12%, ≥ 13%, ≥ 14%, ≥ 15%, or ≥ 16%. This excludes IL2 immunoconjugate preparations made in bacterial cells, which are not glycosylated.

For example, the percentage of glycosylated IL2 immunoconjugate of the total IL2 immunoconjugate in the preparation may be ≥ 2% and ≤ 20%, ≥ 2% and ≤ 19.5%, ≥ 2% and ≤ 19%, or ≥ 2% and ≤ 18.5%. Alternatively, the percentage of glycosylated IL2 immunoconjugate of the total IL2 immunoconjugate in the preparation may be ≥ 5% and ≤ 20%, ≥ 5% and ≤ 19.5%, ≥ 5% and ≤ 19%, or ≥ 5% and ≤ 18.5%. As a further alternative, the percentage of glycosylated IL2 immunoconjugate of the total IL2 immunoconjugate in the preparation may be ≥ 10% and ≤ 20%, ≥ 10% and ≤ 19.5%, ≥ 10% and ≤ 19%, or ≥ 10% and ≤ 18.5%.

Methods for measuring the percentage of glycosylated IL2 immunoconjugate present in a pharmaceutical composition, as well as determining the type of glycosylation present are known in the art and include mass spectrometry, in particular intact mass analysis. An exemplary method is detailed in Example 3. The results of said analysis are shown in Figures 3 to 8.

The pharmaceutical preparation of the invention may comprise, in addition to glycosylated and non-glycosylated IL2 immunoconjugate, as set out in the claims, a pharmaceutically acceptable excipient, carrier, buffer, stabiliser or other materials well known to those skilled in the art and suitable for the administration to human patients. The pharmaceutical composition may comprise a buffer composition as disclosed in WO2018/011404. In particular, the pharmaceutical composition may comprise glycosylated and non-glycosylated IL2 immunoconjugate, as set out in the claims, dissolved in a phosphate buffer comprising NaH₂PO₄ at a concentration of 1-50 mM, NaCl at a concentration of 1-50 mM, KCl at a concentration of 1-2 mM, mannitol at a concentration of 50-200 mM, polysorbate80 at a concentration of 0.05-0.2% (v/v) or of 0.05-0.3%, glycerol at a concentration of 0.5-2% and have a pH from 5.5-7.0. Optionally, the phosphate buffer may further comprise and EDTA at a concentration of 1-20 mM but this is not preferred.

The NaH₂PO₄ may be present at a concentration within a range selected from the group consisting of: 2-25 mM, 3-20 mM, more preferably 5-15 mM. Most preferably the NaH₂PO₄ is present at 6.7 mM or about 6.7 mM. The NaCl may be present at a concentration within a range selected from the group consisting of: 5-40 mM or 10-30 mM or more preferably 15-25 mM. Most preferably NaCl is present at 20 mM or about 20 mM. The KCI may be present at a concentration within a range selected from the group consisting of: 1.2-2.0 mM, more preferably 1.5-1.8 mM. Most preferably, KCl is present at 1.8 mM or about 1.8 mM.

Mannitol may be present at a concentration within a range selected from the group consisting of: 80-180 mM, or more preferably 100-150 mM. Most preferably, mannitol is present at 133 mM or at about 133 mM. Polysorbate80 may be present at a concentration within a range selected from the group consisting of (presented as v/v): 0.1-0.3%, 0.07-0.18%, or more preferably 0.08-0.15%. The polysorbate80 may be present at 0.1% (v/v) or at about 0.1% (v/v). Preferably, polysorbate80 is present at 0.3% (v/v) or at about 0.3% (v/v).

Glycerol may be present at a concentration within a range selected from the group consisting of: 0.7-1.8%, or more preferably 0.8-1.5%. Most preferably, glycerol is present at 1% (w/v) or about 1% (w/v). EDTA may be present at a concentration within a range selected from the group consisting of: 2-15 mM, or more preferably 3-10 mM or 6-9 mM. Most preferably EDTA is present at 5 mM or at about 5 mM. The pH is preferably 6.0-6.8, more preferably 6.1-6.5 mM or 6.2-6.4. Most preferably, the pH is 6.3 or about 6.3. In a preferred embodiment, EDTA is absent.

The pharmaceutical preparation of the invention may comprise, in addition to glycosylated and non-glycosylated IL2 immunoconjugate as set out in the claims, 6.7 mM NaH₂PO₄, 20 mM NaCl, 1.8 mM KCI, 133 mM mannitol, 0.1% polysorbate80 (v/v), 1% glycerol (w/v) and 5mM EDTA and has pH 6.3. Preferably, the pharmaceutical preparation of the invention comprises, in addition to glycosylated and non-glycosylated IL2 immunoconjugate as set out in the claims, 6.7 mM NaH₂PO₄, 20 mM NaCl, 1.8 mM KCI, 133 mM mannitol, 0.3% polysorbate80 (v/v), 1% glycerol (w/v) and has pH 6.3.

Most preferably, the pharmaceutical preparation of the invention comprises, in addition to glycosylated and non-glycosylated IL2 immunoconjugate as set out in the claims, 6.7 mM NaH₂PO₄, 20 mM NaCl, 1.8 mM KCI, 133 mM mannitol, 0.1-0.3% polysorbate80 (v/v), and 1% glycerol (w/v) and has pH 6.3.

Alternatively, the pharmaceutical preparation of the invention may comprise, in addition to glycosylated and non-glycosylated IL2 immunoconjugate as set out in the claims, about 6.7 mM NaH₂PO₄, about 20 mM NaCl, about 1.8 mM KCI, about 133 mM mannitol, about 0.1% polysorbate80 (v/v), about 1% glycerol (w/v) and about 5 mM EDTA and has pH of about 6.3. In a preferred alternative embodiment, the pharmaceutical preparation of the invention may comprise, in addition to glycosylated and non-glycosylated IL2 immunoconjugate as set out in the claims, about 6.7 mM NaH₂PO₄, about 20 mM NaCl, about 1.8 mM KCI, about 133 mM mannitol, about 0.3% polysorbate80 (v/v), about 1% glycerol (w/v) and about 5 mM EDTA and has pH of about 6.3.

As a further alternatively, the pharmaceutical preparation of the invention may comprise, in addition to glycosylated and non-glycosylated IL2 immunoconjugate as set out in the claims, about 6.7 mM NaH₂PO₄, about 20 mM NaCl, about 1.8 mM KCI, about 133 mM mannitol, about 0.1-0.3% polysorbate80 (v/v), and about 1% glycerol (w/v) and has pH of about 6.3.

The preparation of the invention has a higher T cell activation activity compared to a preparation in which the percentage of glycosylated L19-IL2 is 45% or more, preferably 45%, of the total L19-IL2 in the preparation.

As used herein, "T cell activation activity" refers to the ability of an L19-IL2 preparation to enhance T cell proliferation. The T cell activation activity of an L19-IL2 conjugate may be expressed in terms of the half maximal effective concentration (EC50).

The half maximal effective concentration (EC50) is a measure of the effectiveness of a substance in inducing a specific biological or biochemical function. The EC50 is a quantitative measure that indicates how much of an agonist is needed to increase the activity of a given biological process or component of a process such as an enzyme, cell, cell receptor or microorganism by half. Methods of determining EC50 *in vitro* and *in vivo* are known in the art. In the context of the present invention, the EC50 may refer to the amount of L19-IL2 needed to increase the proliferation of a T cell population (e.g. a population of CTLL2 cells) by half.

The preparation of the invention preferably has a half maximal effective concentration (EC50) of at most 4.3 pM, 4.4 pM, 4.5 pM, 5 pM, 5.5 pM, or 6 pM, or a lower EC50. The EC50 of a preparation, such as an L19-IL2 preparation, in the context of enhancing proliferation of a T cell population, can be determined by a T-cell activity assay, such as a CTLL2 T-Cell activity assay e.g., as detailed in Example 5.

In some embodiments, the pharmaceutical preparation of the invention may be administered alone or in combination with other treatments, either simultaneously or sequentially dependent upon the condition to be treated.

Treatments involving the pharmaceutical preparation of the invention may include the administration of a second anti-cancer therapy. Many suitable anti-cancer therapies are known in the art. For example, L19-IL2 has been successfully used in anti-cancer therapy when administered in combination with stereotactic ablative radiotherapy (SABR), or with an anti-PD-1 therapeutic, such as an anti-PD-1 antibody. Clinical trials for these combinations are ongoing. Promising anti-cancer effects have also been reported for the combined administration of L19-IL2 and L19-TNFα (WO2018/011404). This is in addition to the anti-cancer effect demonstrated for L19-IL2 monotherapy.

Thus, the pharmaceutical preparation of the invention may be for use in a method of treating cancer in a patient, wherein the method further comprises administering a second anti-cancer therapeutic selected from the group consisting of: radiotherapy, preferably stereotactic ablative radiotherapy (SABR), a radiolabelled conjugate, preferably a Lutethium-177 labelled conjugate, a check-point inhibitor, such as an anti-PD-1 therapeutic, preferably an anti-PD-1 antibody, L19-TNFα, or L19-TNFα and an anti-PD-1 therapeutic to the patient. Administration of the pharmaceutical preparation of the invention and the second anti-cancer therapy to the patient may be simultaneous or sequential, whereby simultaneous administration refers to administration in the same treatment cycle but not necessarily on the same day.

The pharmaceutical preparation of the invention and the second anti-cancer therapy may be provided as a combined preparation but are preferably provided as separate preparations to permit either simultaneous or sequential administration. Where the treatment further involves radiotherapy, this will necessarily be administered separately from the pharmaceutical preparation of the invention, but administration may nonetheless be simultaneous.

Further treatments may be used in combination with the pharmaceutical preparation of the invention include the administration of suitable doses of pain relief drugs such as non-steroidal anti-inflammatory drugs (e.g. aspirin, paracetamol, ibuprofen or ketoprofen) or opiates such as morphine, or antiemetics.

Where the pharmaceutical preparation of the invention is administered for cancer treatment, it may be injected parenterally. In one embodiment, the pharmaceutical preparation of the invention is injected at the site of the tumour, preferably by intratumoral injection. Peritumoral injection, e.g. local intradermal injection, is another suitable method for administering the pharmaceutical preparation of the invention locally to a tumour site. In some embodiments, the pharmaceutical preparation of the invention may be administered by infusion, e.g. intravenous infusion.

Cancer treatment according to the present invention may include complete eradication of the tumour. The disappearance of any evidence of vital tumour after termination of the treatment represents complete treatment of the tumour. Disappearance of the tumour may be determined when the tumour has no discernible volume or is no longer visible. Treatment may comprise treatment to eradicate the tumour and prevent tumour regrowth.

Patients are preferably monitored during a follow-up period of at least one month, preferably at least six months or at least a year, after administration of the pharmaceutical preparation of the invention. Disappearance of the tumour, and lack of tumour regrowth, may be observed in the follow-up period. Absence of tumour regrowth may be observed.

The quantity of L19-IL2 administered through administration of the pharmaceutical preparation of the invention to the patient will depend on the size and nature of the tumour, among other factors. The determination of suitable doses is within the competence of the skilled practitioner. Suitable doses are disclosed in WO2018/011404. For example, the dose of L19-IL2 may be in the range of 20 µg - 3 mg, e.g. 100-2,500 µg, 300-2,000 µg or between 500-1,800 µg. Preferably, the dose of L19-IL2 is in the range of 2 mg to 3 mg, for example 2 mg to 2.5 mg. Most preferably, the dose of L19-IL2 is 2.17 mg. The dose of L19-IL2 can alternatively be stated in international units (IU). 2.17 mg L19-IL2 equates to 13 million IU L19-IL2. These are examples only and different doses may be used.

The pharmaceutical preparation of the invention may for use in a method of treating cancer. The cancer may be a solid tumor. In a preferred embodiment, the cancer is selected from skin cancer, renal cancer, pancreatic cancer, non-small cell lung cancer, diffuse large B cell lymphoma (DLBCL), basal cell carcinoma, and cutaneous squamous cell carcinoma. The cancer may be metastatic or non-metastatic.

Where the cancer to be treated with the pharmaceutical preparation of the invention is a skin cancer, the pharmaceutical preparation is preferably administered together with L19-TNFα. The L19-TNFα preferably comprises or consists of the sequence set forth in SEQ ID NO: 14. Details of such a therapy are disclosed in WO2013/045125 and in WO2018/01 1404.

**the pharmaceutical** preparation of the invention may be for use in a method of treating skin cancer, such as a malignant melanoma or non-melanoma skin cancer, but preferably a malignant melanoma, in a patient, wherein the method further comprises administering L19-TNFα to the patient. Administration may be by injection at the tumor site, in particular, by intra-tumoral injection. In the case of intra-tumoral injections, patients may be treated with 13 million IU of L19-IL2 corresponding to 2.17 mg of L19-IL2 and with 400 µg of L19-TNFα once weekly for one to four weeks, e.g., for one, two, three or four consecutive weeks. The dose may be administered as a single intra-tumoral injection, or a dose may be divided into multiple intratumoral injections, which are administered to the same tumor. In selected cases the dosage can be halved down to 1.08 mg of L19-IL2 and 200 µg of L19-TNFα.

As used herein, a L19-IL2 preparation comprising a "low percentage" of glycosylated L19-IL2 refers to a preparation in which the percentage of glycosylated L19-IL2 is ≤ 25% of the total L19-IL2 in the preparation.

**As used herein,** a L19-IL2 preparation comprising a "high percentage" of glycosylated L19-IL2 refers to a preparation in which the percentage of glycosylated L19-IL2 is ≥ 40% of the total L19-IL2 in the preparation.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps. It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### Example 1 - Method for producing a preparation of L19-IL2 comprising a low percentage glycosylated L19-IL2

The L19-IL2 production process consisted of a first Upstream phase, dedicated to cell culturing of cells stably expressing L19-IL2, and a second Downstream phase, dedicated to the recovery of the product by chromatographic purification.

In detail, the process consisted of the following steps:
1) Inoculum preparation (Upstream 1),
2) Fermentation (Upstream 2),
3) Purification (Downstream 1 and Downstream 2):
   - Affinity chromatography (Downstream 1);
   - Cation exchange chromatography (CIEX) (Downstream 2);
   - Buffer exchange (diafiltration) (Downstream 2);
   - Formulation (Downstream 2);
   - Anion exchange chromatography (AIEX) (Downstream 2);
   - Nanofiltration (for virus removal) (Downstream 2);
   - Diafiltration (concentration) (Downstream 2).

Process flow diagrams and detailed process descriptions for these manufacturing steps are presented herein.

### Inoculum preparation (Upstream 1) and Fermentation (Upstream 2)

The diagram shown in **Figure 9A** and B describes the inoculum preparation and fermentation processes of L19-IL2 (Upstream 1 and Upstream 2). Upstream 1 and Upstream 2 are divided into several numbered working steps as reported in the following tables.

### Upstream 1 working steps - inoculum preparation

| **Upstream 1** | |
|---|---|
| **Step Nr.** | **Step description** |
| U1-10 | Seeding of L19-IL2 |
| U1-20 | Expansion of L19-IL2 from 1 T75 flask to 1 T150 flask |
| U1-30 | Expansion of L19-IL2 from 1 T150 flask to 4 T150 flask |
| U1-40 | Expansion of L19-IL2 from 4 T150 flasks to 1 roller bottle |
| U1-50 | Expansion of L19-IL2 from 1 roller bottle to 4 roller bottles |
| U1-60 | Expansion of L19-IL2 from 4 roller bottles to 14 roller bottles |
| U1-70 | Preparation of cells for seeding in bioreactor |

### Upstream 2 working steps - fermentation process

| **Upstream 2** | |
|---|---|
| **Step Nr.** | **Step description** |
| U2-10 | Calibration of pH electrode bioreactor |
| U2-20 | Assembly and sterilization of the bioreactor |
| U2-30 | Transfer of culture medium into the bioreactor |
| U2-40 | Calibration pO₂ electrode of the bioreactor |
| U2-50 | Programming fermentation in the bioreactor |
| U2-60 | Inoculation of the bioreactor and start of fermentation (adaptation phase) |
| U2-70 | 1^{st} sterile sampling of the bioreactor |
| U2-80 | 2^{nd} sterile sampling of the bioreactor |
| U2-90 | 3^{rd} sterile sampling of the bioreactor |
| U2-100 | 4^{th} sterile sampling of the bioreactor |
| U2-110 | 5^{th} sterile sampling of the bioreactor |
| U2-120 | Splitting of the bioreactor |
| U2-130 | Production gives "Clarified Bulk" worksheet to warehouse |
| U2-140 | 1^{st} sterile sampling of the bioreactor |
| U2-150 | 2^{nd} sterile sampling of the bioreactor |
| U2-160 | 3^{rd} sterile sampling of the bioreactor |
| U2-170 | 4^{th} sterile sampling of the bioreactor |
| U2-180 | 5^{th} sterile sampling of the bioreactor |
| U2-190 | Splitting of the bioreactor and start of Fed-Batch phase |
| U2-200 | Production gives "Clarified Bulk" worksheet to warehouse |
| U2-210 | 1^{st} sterile sampling of the bioreactor |
| U2-220 | 2^{nd} sterile sampling of the bioreactor |
| U2-230 | 3^{rd} sterile sampling of the bioreactor |
| U2-240 | 4^{th} sterile sampling of the bioreactor |
| U2-250 | 5^{th} sterile sampling of the bioreactor |
| U2-260 | 6^{th} sterile sampling of the bioreactor |
| U2-270 | 7^{th} sterile sampling of the bioreactor |
| U2-280 | 8^{th} sterile sampling of the bioreactor |
| U2-290 | 9^{th} sterile sampling of the bioreactor |
| U2-300 | 10^{th} sterile sampling of the bioreactor |
| U2-310 | 11^{th} sterile sampling of the bioreactor |
| U2-320 | Preparation of Post-Production Cell Bank |
| U2-330 | End of fermentation (Crude Harvest) and sampling |
| U2-340 | Shutdown of the bioreactor |
| U2-350 | Production gives "Clarified Bulk" worksheet to warehouse |
| U2-360 | Lot final summary |

### Inoculum Preparation

The cell culture of one Working Cell Bank vial suitable for the production of L19-IL2 was amplified first in T75/T150 (cm²) square flasks containing 15-30 ml of medium in standard conditions (37°C, 5% CO₂) and then in 1.7 m² roller bottles (37°C, 0.75 rpm) containing 500 ml of medium. The cell culture was expanded up to a volume of 7 liters and to a cell concentration higher than 900.000 cells/ml with a viability higher than 70%. CD Hybridoma medium (ThermoFisher cat n. 11279023), supplemented with 4 mM Glutamine, was used for square flask cell culturing, while 20 mM HEPES buffer was added for cell culturing in roller bottles. At the end of the amplification, the cell suspension was transferred into a sterile bag using a peristaltic pump and connected to the bioreactor using a steam-in-place stainless steel connector.

### Fermentation

Fermentation took place in a Biostat C 30 L bioreactor sterilized *in situ* and equipped with a gas mixer and a ring sparger aeration system. The pH was controlled by a flow of CO₂ and calibrated additions of sodium carbonate solution. The agitation system was composed of a single marine impeller. The temperature was controlled by a water jacket connected to a steam heat exchanger. The working temperature was 37±0.01°C. The concentration of the dissolved oxygen was maintained at a 38% ± 2% air saturation value with a flow of air, nitrogen, and oxygen mixture. The relative ratios of these three different gasses were controlled by the system management software through the gas mixer device of the bioreactor. The partial pressure of each of the three gases was 0.95 atm. Cell culture aeration was performed through use of a ring sparger diffusion system. The maximum gas mixture flow rate was 1.1 slpm ± 0.05. Before the addition of the inoculum, the bioreactor was aseptically filled with 14 L of culture medium (protein-free, chemically defined CD Hybridoma Medium + 6 mM Glutamax). The medium, contained in sterile plastic bags, was transferred into the bioreactor vessel by gravity through the steam-in-place sterilized addition port device. The fermentation process consisted of a semi-continuous cell adaptation period followed by fed-batch fermentation.

### Cell adaptation

The cell adaptation period started when the inoculum (7 L of cell culture in a sterile plastic bag) was added to the fresh medium in the bioreactor. During this period, the cell culture was added with glucose and L-ultra-glutamine at predefined time intervals and concentrations. The stirring speed was set at 80±5rpm. After 3-5 days, the bioreactor was split 1:4, the harvest was discarded and replaced with the same volume of fresh medium. After 3-5 days, the cell culture was split again, this time with a ratio 1:5. 4.2 L of cell culture were left in the bioreactor, and supplemented with 16.8 L of fresh medium to reach an overall volume of 21 L. This passage closed the cell adaptation period. At the end of the cell adaptation period, the cell count typically reached a viable cell concentration between 3-4,000,000 cells/ml, with a productivity of 30-40 mg/L. The cell culture harvested during this phase was discarded without any further processing.

### Fed-batch Fermentation

The fed-batch fermentation started at the second cell culture splitting, through addition of 16.8 L of fresh medium to 4.2 L of cell culture used as inoculum, with a 1:5 dilution factor. The stirring speed was set at 88±5rpm.The fed-batch fermentation lasted 10-11 days (depending on the cell viability); during this period, the cell culture was fed at regular intervals with both a solution of HyClone Cell Boost 3 35 g/L in Pro CHO5 medium, and L-ultraglutamine 200 mM. The approximate feed pattern is reported in the following table.

| | **Time of fermentation** | | | | | | | |
|---|---|---|---|---|---|---|---|---|
| **Feed** | **74h - 84h** | **100h** | **130h** | **160h** | **180h** | **200h** | **220h** | **240h** |
| Cell Boost (ml) | 3120 | 2185 | 1250 | 1250 | 1250 | 1250 | 1250 | 1250 |
| L-Ultra Glutamine (ml) | 75 | 75 | 75 | 75 | 75 | 75 | 75 | 75 |

During the fed-batch fermentation, the cell culture was aseptically sampled daily. At the end of the process, the total volume of solution added was approximately 10-12 L, therefore a volume of approximately 30-33 L of cell culture was harvested from the bioreactor. At the end of the fed-batch fermentation, the final harvest generally had ~5,000,000 viable cells/ml with a productivity of ~150 mg/L secreted protein in the medium. The process described above can also be performed using two twin bioreactors Biostat C 30L. In this case, 4.2 L of the cell culture from the 1:5 splitting of the first bioreactor are used as inoculum for the second one. As the cells of the inoculum are already adapted to the fermentation conditions, the semi-continuous cell adaptation period is not required for the second bioreactor, and the fed-batch fermentation can run simultaneously with the first bioreactor. At the end of the fed-batch fermentation, the Crude Harvest was collected, and cells were removed by dead end filtration (see "Microfiltration" below). A complete fermentation run lasted a total of 18 days, excluding the washing and sterilization of the equipment, and produced a total harvest of ~ 60-65 L for both bioreactors. Typically, 8 to 10 g of crude harvest were obtained from a fermentation run.

### Microfiltration

The Crude Harvest was clarified and sterilized with orthogonal filtration on single-use cartridge filters. The clarification step was carried out with a Sartopure GF2, 0.65 µm, 0.6 m² cartridge filter, while further clarification and sterilization was obtained through a combined single-use SartobranP, 0.45/0.22 µm, 0.45 m² cartridge filter. The harvested material was loaded into the filters with a peristaltic pump at a flow rate of 1-2 L/min. After filtration, 0.5 M EDTA solution pH 7.5 was added to the harvested material to reach a final EDTA concentration of 5 mM, obtaining the Clarified Bulk. The Clarified Bulk was stored at 2-8°C for a maximum of 40 days until the next purification step.

### Purification (Downstream 1 and Downstream 2)

The diagram shown in Figure 10A and B show the purification process steps used to obtain L19-IL2 (Downstream 1 and Downstream 2).

Downstream 1 and Downstream 2 are divided into several numbered working steps as reported below.

### Downstream 1 working steps

| **Downstream 1** | |
|---|---|
| **Step Nr.** | **Step description** |
| D1-10 | Preparation of the Protein A rmp Sepharose Fast Flow purification system |
| D1-20 | Purification of Clarified Bulk on rmpProtein A resin Sepharose Fast Flow, 1^{st} run |
| D1-30 | Purification of Clarified Bulk on rmpProtein A resin Sepharose Fast Flow, 2^{nd} run |
| D1-40 | Purification of Clarified Bulk on rmpProtein A resin Sepharose Fast Flow, 3^{rd} run |
| D1-50 | Purification of Clarified Bulk on rmpProtein A resin Sepharose Fast Flow, 4^{th} run |
| D1-60 | Purification of Clarified Bulk on rmpProtein A resin Sepharose Fast Flow, 5^{th} run |
| D1-70 | Purification of Clarified Bulk on rmpProtein A resin Sepharose Fast Flow, 6^{th} run |

### Downstream 2 working steps.

| **Downstream 2** | |
|---|---|
| **Step Nr.** | **Step description** |
| D2-10 | Preparation of the purification system on Source 30S |
| D2-20 | Request of "Semiprocessed" to the warehouse |
| D2-30 | 1^{st} purification run by Source 30 S |
| D2-40 | 2^{nd} purification run by Source 30 S |
| D2-50 | 3^{rd} purification run by Source 30 S |
| D2-60 | Shutdown of purification system |
| D2-70 | Preparation of the Sartocon Slice cassette system |
| D2-80 | Buffer Exchange through the Sartocon system |
| D2-90 | Formulation |
| D2-100 | Shutdown of Buffer Exchange system |
| D2-110 | Purification of the Purification Intermediate 2 by Sartobind Q |
| D2-120 | Cleaning in place (CIP) system |
| D2-130 | Nanofiltration of the Purification Intermediate 3 |
| D2-140 | Concentration of Purification Intermediate 4 |
| D2-150 | Purified Bulk to the warehouse and CIP system |

Downstream 1 and Downstream 2 steps required the preparation of several buffers used for the sanitization, equilibration, elution and washing of the columns. Each buffer was prepared in a stainless-steel tank under mechanical stirring, and the pH was adjusted with 5 M NaOH or 5 M HCl. Buffers were then sterile filtered (by Sartolon filters, 0.22 µm, 0.2m2) and transferred into sterile bags or bottles by a peristaltic pump.

### Affinity Chromatography on Protein A (Downstream 1)

Protein A affinity chromatography was performed using a rmp Sepharose Fast Flow resin packed in a Vantage A-130 or A-180 column. The rmp Sepharose Fast Flow resin was a low leakage, non-mammalian-based affinity resin designed for high purity separation of monoclonal and polyclonal antibodies. The base matrix, Sepharose 4 Fast Flow, was a highly cross-linked, 4% agarose derivative, containing five antigen binding domains. The scFv (L19) moiety of L19-IL2 fusion protein binds to protein A immobilized on the resin. The Vantage A-130 and the Vantage A-180 columns differ in the volume of resin which can be packed into the column, from 400mL to 700mL for Vantage A-130 and from 700mL to 1300 mL for Vantage A-180. Before loading the sample, the column, resin and flow line of the chromatographic system were sanitized in place with a solution of Acetic acid 0.1 M / Ethanol 20 %. The contact time was 60 minutes. After the treatment, the system was equilibrated in PBS (NaH2PO4·H2O 50 mM; NaCl 150 mM, EDTA 5 mM, pH 7.5). Typically, 1000 mL of rmp Sepharose FF-Protein A resin was used with a Vantage 180 column. Up to 4.0g of crude L19-IL2, typically contained in 80-100L of Clarified Bulk, were loaded at room temperature onto the column at 100 ml/min. Once the sample was loaded, the resin was washed at a flow rate of 150-200 ml/min. The washing buffers and the volume applied were:
- 5.0 L (5 CV) of PBS/NaCl (NaH₂PO4·H₂O 50 mM; NaCl 650 mM, EDTA 5 mM, pH 7.5).
- 5.0 L (5 CV) of Gly/NaCl (Glycine 100 mM; NaCl 150 mM, EDTA 5 mM, pH 4.0)

The L19-IL2 fusion protein was then eluted from the column with Gly/NaCl (Glycine 100 mM; NaCl 150 mM, EDTA 5 mM, pH 3.0). The chromatographic run was conducted by monitoring the absorbance at 280 and 260 nm, conductivity, and pH. The eluted sample underwent viral inactivation through incubation at pH 3.0 for 1 hour at room temperature. Upon incubation, glycerol to 1% w/v of final concentration was added (conditioning), obtaining the Semi-processed product. The O.D. (280nm) at this stage ranges between 1.0 and 1.7. The Semi-processed product was stored at -80°C.

### Cation Exchange Chromatography (CIEX) (Downstream 2)

The Cation Exchange Chromatography step separated the main L19-IL2 isoforms: monomer, homodimer, and trimer. The form of interest was represented by the non-covalent homodimeric form of L19-IL2. Before loading the sample on the column, resin and flow line of the chromatographic system were sanitized in place with a solution of Sodium hydroxide 1M for 60 minutes. After sanification, the system was equilibrated in buffer "A" (Na2SO4 15 mM; Na2HPO4·7H2O 15 mM, EDTA 5 mM, pH 6.00, 4.50 - 6.50 mS/cm). Purification was carried out on Source 30S packed in a Vantage A2-60 column. Flow rate was 300 cm/h corresponding to 150 ml/min. The Semi-processed was thawed in a water bath at room temperature. The ionic strength was regulated to 5-6 mS/cm through dilution 4:5 with Conditioning Solution (Na2SO4 100 mM; Na2HPO4·7H2O 100 mM, EDTA 5 mM, pH 7.20, 23.50 - 27.50 mS/cm), followed by a further dilution with water for injection 1:4. The pH was corrected to 6.00 ± 0.10. The product was then filtered with a cartridge filter SartobranP, 0.22 µm, 0.05 m2 (or 0.1 m2 according to the volume) into sterile plastic bags and loaded on the column at room temperature. The column was then washed at 150 ml/min with 2 CV (2 litres) of buffer "A" (Na2SO4 15 mM; Na2HPO4·7H2O 15 mM, EDTA 5 mM, pH 6.00). The elution of the various L19-IL2 isoforms was carried out at 150 ml/min with a continuous saline gradient, using two buffer solution: Buffer "A" (Na2SO4 15 mM; Na2HPO4·7H2O 15 mM, EDTA 5 mM, pH 6.00, 4.50 - 6.50 mS/cm) and Buffer "B" (NaCl 500 mM, Na2SO4 15 mM, Na2HPO4·7H2O 15 mM, EDTA 5 mM, pH 6.00, 44.0 - 48.0 mS/cm). The gradient applied can be divided into 2 different phases: 1: 0-40% B in 10 CV and 2: 40-100% B in 0 CV.

The various L19-IL2 isoforms (monomer, homodimer, and trimer) eluted respectively at 9.5 - 14.0 mSi/cm (monomer), 15.0 - 19.0 mSi/cm (dimer), 19.0.- 21.0 mSi/cm (trimer). The relative ratio of the various isoforms was usually in the range of 25%-35% monomer, 55%-65% homodimer and 3%-10% trimer. The comprehensive yield for this step was 35%-45% considering the initial total amount of L19-IL2, and higher than 75% considering only the initial amount of homodimeric form of L19-IL2. L19-IL2 purified homodimer (Purification Intermediate 1) was filtered through a 0.22 µm sterile filter in glass bottles and stored at 2-8°C until the next process step. When all the runs of a batch were completed, the Purification Intermediates 1 were pooled before being processed by buffer exchange.

### Buffer Exchange (Downstream 2)

The buffer exchange process was performed using the Sartocon Slice cassette system (30kDa), a tangential filtration system based on the hydrophilic and neutral cellulose-based membrane Hydrosart with 30kDa cut-off. This system permits the transfer of L19-IL2 to the formulation buffer using several cycles of diafiltration. Before use, the cassette was washed with a 0.9% NaCl solution for 10 minutes and sanitized with a NaOH 1M solution, recirculated for not less than 60 minutes, at 1.5 bar pressure. After sanitization, the cassette was washed with 1 L of WFI, then equilibrated with formulation buffer (6.7 mM NaH2PO4, 1.8 mM KCI, 20 mM NaCl, 133 mM Mannitol, 1% w/v Glycerol, pH 6.30) until pH and conductivity of the permeate match the values of the formulation buffer. When the cassette was equilibrated, the diafiltration could start. The permeate outlet was connected to the UV/pH/conductivity monitors on the ÄKTA Pilot System, then the inlet and the retentate outlet were connected to the three ways cap of the sample bottle. The third inlet of the sample bottle cap was connected to the buffer supply bottle. The Purification Intermediate 1 passes through the slice cassette: the protein was retained and returns to the starting bottle (retentate), while the elution solution flows away as permeate. At the same time, the volume of permeate was replaced by a same amount of formulation buffer. When the conductivity and pH of retentate reach the same values as formulation buffer, the buffer exchange was completed and the Purified Sample was obtained.

### Formulation (Downstream 2)

A solution 0.1% v/v Tween80 was then added to the purified sample. After this formulation step, the Purification Intermediate 2 was obtained (optical density comprised between 0.800 and 1.000).

### Anion Exchange Chromatography (AlEX) (Downstream 2)

The Anion Exchange Chromatography purification step was performed on Sartobind Q cartridge. It was a strong basic anion exchanger based on quaternary ammonium. Before loading the sample, the capsule was activated by pumping at least 200 ml of NaCl 1M, then NaOH 1M was flushed for at least 60 minutes to sanitize the capsule (supplied as non-sterile). Sartobind Q capsule was used in flow through mode, so that the L19-IL2 does not bind to the membrane and it was collected directly into the flow through container. The DNA potentially present in the sample was removed by binding to the Sartobind Q matrix. This chromatography resulted in the Purification Intermediate 3.

### Nanofiltration (Downstream 2)

The purpose of the nanofiltration process was to remove any virus from the Purification Intermediate 3. The nanofiltration was performed with a Pro Modus 1.1 or Pro Modus 1.2 Viresolve filter. The filter was flushed with water for 10 minutes, sanitized flushing NaOH 0.5M for 1 hour and then equilibrated with formulation buffer. The filtration flow rate was automatically adjusted by the ÄKTA Pilot system to keep a constant pressure. The filtered material represents the Purification Intermediate 4 with an optical density between 0.800 and 1.000.

### Diafiltration (Downstream 2)

The purpose of the diafiltration process was to concentrate the Purification Intermediate 4 to reach an optical density between 2.100 and 2.400. The concentration was performed using a 30kDa Sartocon slice cassette system. The system can concentrate the Purification Intermediate 4 through several cycles of diafiltration; in this case the concentration factor was around 3. In this process the formulation buffer filtered through the slice cassette (permeate) was not replaced, while the protein was retained and returned to the starting bottle (retentate). Before use, the cassette was washed with a 0.9% NaCl solution for 10 minutes and sanitized with a NaOH 1M solution, recirculated for at least 60 minutes, at 1.5 bar pressure. After sanitization, the cassette was washed with 1L of WFI, then equilibrated with formulation buffer until pH and conductivity of the permeate match the values of formulation buffer. When the cassette was equilibrated, the concentration can start. The permeate outlet was connected to the UV/pH/Conductivity monitors on the ÄKTA Pilot System, then the Inlet and the Retentate Outlet were connected to the three ways cap of the Sample Bottle. The A280nm of the retentate was checked in process; the concentration process was stopped when the A280nm reaches a value of 2.250 ± 0.150. The Purified Bulk, obtained at the end of this process, represented the Drug Substance. During this concentration step, also the excipient Polysorbate 80 was concentrated to 0.3% v/v.

### Example 2 - Method for producing a preparation of L19-IL2 comprising a high percentage glycosylated L19-IL2 (not according to the claimed invention; included for comparative purposes only)

L19-IL2 was expressed by transient gene expression using the mammalian expression vector pcDNA3-L19-IL2 according to a standard method described below (i.e., not according to the claimed invention).

For 1 ml of production, 4 × 10⁶ cells were collected by centrifugation and resuspended in 1 mL of medium supplemented with 4 mM ultraglutamine. 0.75 µg of plasmid DNAs followed by 2.5 µg polyethylene imine (PEI; 1 mg/mL solution in water at pH 7.0) per million cells, were added to the cells and gently mixed. The transfected cultures were incubated in a shaker incubator at 31°C for 6 days.

L19-IL2 was then purified by Protein-A affinity chromatography followed by a polishing step by Cation Exchange Chromatography (CIEX). Briefly, the six days old supernatant from transfected cells was clarified by centrifugation and filtration before loading on HiTrap^{™} rProtein A Fast Flow column using an ÄKTA PURE chromatographic system. The column was washed by 5 consecutive steps consisting of (1) 150mM NaCl, 50mM NaH₂PO₄, 5mM EDTA, 50mM NaOH, pH 7.5 (2) 650mM NaCl, 50mM NaH₂PO₄, 5mM EDTA, 53mM NaOH, pH 7.5 (3) 50 mM NaH₂PO₄, 150 mM NaCl, 1M Urea, 5 mM EDTA, 10% (v/v) Isopropanol, pH 7.5 (4) 250mM L-Arginine, pH 7.5 (5) 50mM Citric acid, pH4, before eluting the L19-IL2 sample with 50mM Citric acid, pH4. The collected eluate was then further purified by CIEX using a Source 30S resin as solid support.

Briefly, the Protein-A purified L19-IL2 sample was conditioned by dilution with 2.5 volumes of MilliQ-H₂O and 1.15 volumes of 100mM Na₂SO₄, 100mM Na₂HPO₄, 5mM EDTA, pH 7.2, before loading on a Source 30S column. The loaded L19-IL2 was then eluted using a 0-40% gradient from Buffer A (15mM Na₂SO₄, 15mM Na₂HPO₄, 5mM EDTA, pH6) to Buffer B (15mM Na₂SO₄, 15mM Na₂HPO₄, 5mM EDTA, 0.5M NaCl, pH6). Finally, the polished L19-IL2 sample was aliquoted, snap-frozen in liquid nitrogen and stored at -80°C till further use.

### Example 3 - Quality Control of L19-IL2

Purity of the L19-IL2 lots prepared according to Example 1 were characterized using several quality control analyses, including ELISA, SDS-PAGE, Size Exclusion Chromatography, A₂₈₀ measurements, productivity, bioactivity and immunoreactivity assays, osmolality, viral testing, visual appearance etc. The protocol used for 2D-SDS-PAGE analysis, is reported below.

### 2D-SDS-PAGE

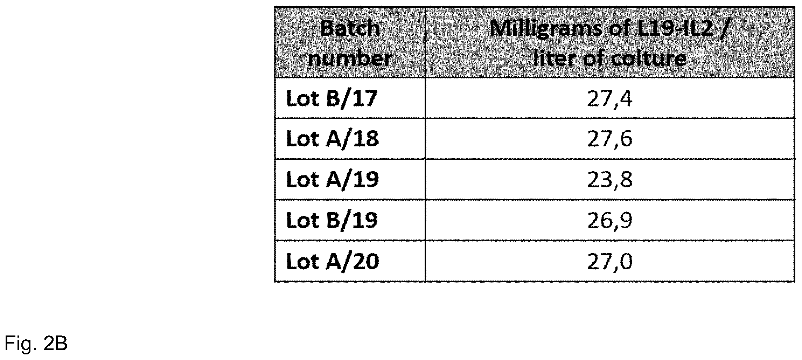

Each sample was analyzed in bidimensional electrophoreses assay using a precast system (ZOOM^{®} IPGRunner^{™}). Proteins were separated on the basis of their molecular weight and isoelectric point. Staining was performed with Blue Coomassie R-250. The results are reported in **Figure 2****.**

### Example 4 - Mass Spectrometry analysis of L19-IL2

### Direct Infusion HR-MS for intact mass analysis

100 µg of the various L19-IL2 samples were desalted using C18-purification before analysis (Micro Spin Columns, Harvard Apparatus). Direct infusion was performed on an Orbitrap Q-Exactive coupled to an Ion Max ESI source. The following parameters were used: syringe flow rate 4 µL/min, capillary voltage 3.0 kV, in source induced dissociation 40 eV, sheath gas 4 units, capillary temperature 300 °C, S-lens RF level 90, resolution 17500 (FWHM at 200 m/z), AGC target 5 x 10⁴, microscan 10, mass range 500-3000 m/z, and maximum injection time 200 ms. Raw spectra were then analyzed to quantify relative amounts of glycosylated and non-glycosylated L19-IL2 of the total L19-IL2 in the preparation. Quantification was achieved by summing the intensities of the peaks corresponding to the glycosylated and non-glycosylated L19-IL2 species, and the percentage was calculated using the equation below. $\frac{Intensity of x}{\sum Intensities} \times 100$

Where x is the sum of the intensities of a single L19-IL2 variant (non-glycosylated, HexNAc1Hex1NeuGc1 glycosylated, or HexNAc1Hex1NeuGc2 glycosylated). The results of this analysis are reported in **Figures 3** and **4****.** These figures illustrate that the percentage of glycosylated L19-IL2 in the preparation prepared according to the method described herein **(****Figures 3A** and **4A****)** is significantly lower than the percentage of glycosylated L19-IL2 in the preparation prepared according to a standard method **(****Figures 3B** and **4B****).**

### MS/MS analysis for glycopeptide analysis

7.5 µg of L19-IL2 were resuspended in Urea 1 M dissolved in NH₄HCO₃ solution at a final pH = 8. Protein was reduced with TCEP for 15 min at RT followed by 45 min at 65 °C and alkylated with lodoacetamide (IAA) for 1 hour in the dark. Protein was then digested by trypsin (enzyme-protein ratio 1:60) at 37 °C overnight. After digestion, the sample was acidified with 10% formic acid and then subjected to C18 purification and desalting (Macro Spin Columns, Harvard Apparatus). 500 ng of the resulting peptides were then subjected to HPLC-MS/MS analysis. All samples were analyzed on an Orbitrap Q-Exactive mass spectrometer coupled to an EASY nanoLC 1000 system via a Nano Flex ion source. Chromatographic separation was carried out on an Acclaim PepMap RSLC column (50 µm x 15 cm, particle size 2 µm, pore size, 100 Å, using 40 min linear gradient with 5-35% solvent B (0.1% formic acid in acetonitrile) at a flow rate of 300 nL/min. Ionization was carried out in positive ion mode, with 2 kV of spray voltage, 250 °C of capillary temperature, 60 S-lens RF level. The mass spectrometer was working in a data-dependent mode. MS1 scan range was set from 350 to 1650 m/z, the 10 most abundant peptides were subjected to HCD fragmentation with NCE of 25. A dynamic exclusion was set at 10 seconds. Raw files were processed with Proteome Discoverer 1.4. Database searches were performed using Sequest as search engine using a FASTA file containing our protein of interest, the *mus musculus* reference proteome and additional contaminants (human keratin isoforms, bovine serum albumin and ProteinA from *Staphylococcus Aureus).* Carbamidomethylation of cysteines was set as a fixed modification while oxidation of methionine and different O-glycosylations (HexNAc1Hex1NeuGc1, HexNAc1Hex1NeuGc2) were set as variable modifications. Trypsin was set as cleavage specificity, allowing a maximum of 2 missed cleavages. Data filtering was performed using percolator, resulting in 1% false discovery rate (FDR). The results of this analysis are shown in **Figures 5** to **8****.**

### Example 5 - CTLL2 T-Cell activity assay

To show the effect of glycosylation level on T cell activation activity, the preparations comprising a high percentage of glycosylated L19-IL2 were compared with those comprising a low percentage of glycosylated L19-IL2 in a T cell proliferation assay. The assay is based on a colorimetric method for determining the number of viable cells in proliferation.

### Cell Culture

The CTLL-2 cell line is a clone of cytotoxic T cells which are IL2 dependent for growth, and which are routinely used to assay the activity of IL2 and IL2 conjugates such as L19-IL2. CTLL2 cells were grown in RPMI-1640 medium (Gibco; 21875-034), supplemented with 10% FBS and 2mM L-Glutamine. Fresh 10% T-STIM with ConA was added at each passage. Cells were cultured in 20mL culture medium in a T-75 flask maintained at 37°C and 5%CO₂. Cells were kept at a density between 0.1-0.5mio/mL.

### Day 1:

Step 1: Cells were counted (e.g., 20mL at 0.4mio/mL), spun for 5 minutes at 900rpm, and then washed with 50mL of culture medium.
Step 2: Cells were spun for 5 minutes at 900rpm, and then washed with 50mL of culture medium.
Step 3: Cells were spun for 5 minutes at 900rpm, before being resuspended at the initial concentration in 20mL of culture medium.
Step 4: Cells were incubated at 37°C and 5%CO₂ for 24 hours.

### Day 2:

Step 1: Cells were spun for 5 minutes at 900rpm, and then washed with 50mL of culture medium.
Step 2: Cells were spun for 5 minutes at 900rpm, before being resuspended in culture medium at a concentration of 0.5mio/mL.
Step 3: 50µL of cells were added per well (i.e., 25'000 cells/well) in a 96-well plate.
Step 4: 50µL of protein samples, diluted in culture medium according to a serial dilution scheme, were added to each well.
Step 5: Cells were incubated at 37°C and 5%CO₂ for 48 hours.

### Day 4:

Step 1: 20µL of CellTiter Aqueous One Solution was added per well and the cells were incubated at 37°C and 5%CO₂.
Step 2: Absorbance was measured at 490nm vs 620nm at the following timepoints: 1h, 2h, 3h and 4h.

### Data normalization:

Step 1: The medium only background was subtracted from all samples' absorbance data.
Step 2: Data was normalized using the formula: (sample - average cells only) / average cells only.

### Results:

The results of this assay are shown in Figure 11, which illustrates that the L19-IL2 preparation comprising a low percentage of glycosylated L19-IL2 shows a superior T cell activation activity (EC50 4.3 pM) as compared to the L19-IL2 preparation comprising a high percentage of glycosylated L19-IL2 (EC50 9.5 pM).

### Sequence Listing

### Amino acid sequence of L19 CDRs

L19 CDR1 VH -SFSMS (SEQ ID NO: 1)
L19 CDR2 VH -SISGSSGTTYYADSVKG (SEQ ID NO: 2)
L19 CDR3 VH -PFPYFDY (SEQ ID NO: 3)
L19 CDR1 VL -RASQSVSSSFLA (SEQ ID NO: 4)
L19 CDR2 VL -YASSRAT (SEQ ID NO: 5)
L19 CDR3 VL -QQTGRIPPT (SEQ ID NO: 6)
Amino acid sequence of the L19 VH domain (SEQ ID NO: 7)
Amino acid sequence of the linker between VH and VL (SEQ ID NO: 8) GDGSSGGSGGAS
Amino acid sequence of the L19 VL domain (SEQ ID NO: 9)
Amino acid sequence of the L19 scFv (SEQ ID NO: 10) 
EVQLLESGGGLVQPGGSLRLSCAASGFTFSSFSMSWVRQAPGKGLEWVSSISGSSGTTYYAD
SVKGRFTISRDNSKNTLYLQMNSLRAEDTAVYYCAKPFPYFDYWGQGTLVTVSSGDGSSGGS
GGASEIVLTQSPGTLSLSPGERATLSCRASQSVSSSFLAWYQQKPGQAPRLLIYYASSRATGIP
DRFSGSGSGTDFTLTISRLEPEDFAVYYCQQTGRIPPTFGQGTKVEIK
Amino acid sequence of human Interleukin 2 (hulL-2) (SEQ ID NO: 11). *Threonine 3 is shown in bold, italics and underlined*
Amino acid sequence of the linker between scFv and IL2 (SEQ ID NO: 12) EFSSSSGSSSSGSSSSG
Amino acid sequence of the L19-IL2 conjugate (SEQ ID NO: 13) *Threonine* 256 *is shown in bold, italics and underlined* 
EVQLLESGGGLVQPGGSLRLSCAASGFTFSSFSMSWVRQAPGKGLEWVSSISGSSGTTYYAD
SVKGRFTISRDNSKNTLYLQMNSLRAEDTAVYYCAKPFPYFDYWGQGTLVTVSSGDGSSGGS
GGASEIVLTQSPGTLSLSPGERATLSCRASQSVSSSFLAWYQQKPGQAPRLLIYYASSRATGIP
DRFSGSGSGTDFTLTISRLEPEDFAVYYCQQTGRIPPTFGQGTKVEIKEFSSSSGSSSSGSSSS
GAPTSSSTKKTQLQLEHLLLDLQMILNGINNYKNPKLTRMLTFKFYMPKKATELKHLQCLEEELK
PLEEVLNLAQSKNFHLRPRDLISNINVIVLELKGSETTFMCEYADETATIVEFLNRWITFCQSIIST
LT
Amino acid sequence of the L19-huTNFα conjugate (SEQ ID NO: 14) *Serine* 257 *is shown in bold, italics and underlined*

EVQLLESGGGLVQPGGSLRLSCAASGFTFSSFSMSWVRQAPGKGLEWVSSISGSSGTTYYAD
SVKGRFTISRDNSKNTLYLQMNSLRAEDTAVYYCAKPFPYFDYWGQGTLVTVSSGDGSSGGS
GGASEIVLTQSPGTLSLSPGERATLSCRASQSVSSSFLAWYQQKPGQAPRLLIYYASSRATGIP
DRFSGSGSGTDFTLTISRLEPEDFAVYYCQQTGRIPPTFGQGTKVEIKEFSSSSGSSSSGSSSS
GVRSSSRTPSDKPVAHVVANPQAEGQLQWLNRRANALLANGVELRDNQLVVPSEGLYLIYSQ
VLFKGQGCPSTHVLLTHTISRIAVSYQTKVNLLSAIKSPCQRETPEGAEAKPWYEPIYLGGVFQL
EKGDRLSAEINRPDYLDFAESGQVYFGIIAL

## Claims

1. A pharmaceutical preparation comprising:
(i) non-glycosylated L19-IL2; and
(ii) glycosylated L19-IL2;
wherein the L19-IL2 comprises or consists of the sequence set forth in SEQ ID NO: 13, and
wherein the percentage of glycosylated L19-IL2 is ≤ 20% of the total L19-IL2 in the preparation.

2. The pharmaceutical preparation of claim 1, wherein the glycosylated L19-IL2 comprises an *O*-linked glycosylation on the threonine at position 256 of SEQ ID NO: 13.

3. The pharmaceutical preparation of claim 2, wherein the *O*-linked glycosylation consists of HexNAc₁Hex₁NeuGc₁ or HexNAc₁Hex₁NeuGc₂.

4. The pharmaceutical preparation according to any one of claims 1 to 3, further comprising a pharmaceutically acceptable excipient, carrier, buffer, stabiliser.

5. The pharmaceutical preparation according to any one of claims 1 to 4, for use in a method of treating cancer in a patient.

6. The pharmaceutical preparation for use according to claim 5, wherein the cancer is skin cancer, renal cancer, pancreatic cancer, non-small cell lung cancer, diffuse large B cell lymphoma (DLBCL), basal cell carcinoma, or cutaneous squamous cell carcinoma.

7. The pharmaceutical preparation for use according to claim 5 or 6, wherein the method comprises administering a second anti-cancer therapeutic to the patient.

## Patentansprüche

1. Pharmazeutisches Präparat, das Folgendes umfasst:
(i) nichtglykosyliertes L19-IL2 und
(ii) glykosyliertes L19-IL-2;
wobei das L19-IL2 die in SEQ ID NO: 13 dargelegte Sequenz umfasst oder daraus besteht und wobei der Prozentsatz von glykosyliertem L19-IL2 ≤ 20 % des Gesamt-L19-IL2 in dem Präparat beträgt.

2. Pharmazeutisches Präparat nach Anspruch 1, wobei das glykosylierte L19-IL2 eine O-gebundene Glykosylierung auf dem Threonin an Position 256 von SEQ ID NO: 13 umfasst.

3. Pharmazeutisches Präparat nach Anspruch 2, wobei die O-gebundene Glykosylierung aus HexNAc₁Hex₁NeuGc₁ oder HexNAc₁Hex₁NeuGc₂ besteht.

4. Pharmazeutisches Präparat nach einem der Ansprüche 1 bis 3, das weiters einen pharmazeutisch annehmbaren Hilfsstoff, Träger, Puffer oder Stabilisator umfasst.

5. Pharmazeutisches Präparat nach einem der Ansprüche 1 bis 4 zur Verwendung in einem Verfahren zur Behandlung von Krebs in einem Patienten.

6. Pharmazeutisches Präparat zur Verwendung nach Anspruch 5, wobei der Krebs Hautkrebs, Nierenkrebs, Bauchspeicheldrüsenkrebs, nichtkleinzelliger Lungenkrebs, diffuses großes B-Zelllymphom (DLBCL), Basalzellkarzinom oder kutanes Plattenepithelkarzinom ist.

7. Pharmazeutisches Präparat zur Verwendung nach Anspruch 5 oder 6, wobei das Verfahren das Verabreichen eines zweiten Anti-Krebs-Therapeutikums an den Patienten umfasst.

## Revendications

1. Préparation pharmaceutique, comprenant :
(i) de la L19-IL2 non glycosylée ; et
(ii) de la L19-IL2 glycosylée ;
dans laquelle la L19-IL2 comprend ou consiste en la séquence présentée dans SEQ ID NO : 13, et dans laquelle le pourcentage de L19-IL2 glycosylée est ≤ 20 % de la L19-IL2 totale dans la préparation.

2. Préparation pharmaceutique selon la revendication 1, dans laquelle le L19-IL2 glycosylé comprend une glycosylation liée en *O* sur la thréonine en position 256 de SEQ ID NO : 13.

3. Préparation pharmaceutique selon la revendication 2, dans laquelle la glycosylation liée en *O* consiste en HexNAC₁Hex₁NeuGc₁ ou HexNAC₁Hex₁NeuGc₂.

4. Préparation pharmaceutique selon l'une quelconque des revendications 1 à 3, comprenant en outre un excipient, un support, un tampon, un stabilisant pharmaceutiquement acceptable.

5. Préparation pharmaceutique selon l'une quelconque des revendications 1 à 4, pour utilisation dans un procédé de traitement du cancer chez un patient.

6. Préparation pharmaceutique destinée à être utilisée selon la revendication 5, dans laquelle le cancer est un cancer de la peau, un cancer du rein, un cancer du pancréas, un cancer du poumon non à petites cellules, un lymphome diffus à grandes cellules B (DLBCL), un carcinome basocellulaire ou un carcinome épidermoïde cutané.

7. Préparation pharmaceutique pour utilisation selon la revendication 5 ou 6, dans laquelle le procédé comprend l'administration d'un second agent thérapeutique anticancéreux au patient.
